# EUROPEAN PATENT APPLICATION

(11) **EP 0 997 470 A1**
(43) Date of publication of application: **03.05.2000**
(21) Application number: 99121580.7
(22) Date of filing: 29.10.1999
(51) Int. Cl.: C07F 9/50, C07F 15/00, C07B 53/00, C07C 67/303, C07C 29/143, C07C 45/69

(54) **Optically active bisphosphinomethanes and their rhodium and copper complexes for catalytic asymmetric syntheses**

(30) Priority: 29.10.1998 JP 30897798
(71) Applicant: Chiba University, Chiba-City, Chiba Pref. (JP)
(72) Inventor: Imamoto, Tsuneo, Chiba City, Chiba Pref. (JP); Yamanoi, Yoshinori, Kawaguchi City, Saitama Pref. (JP)
(74) Representative: Henkel, Feiler, Hänzel

(57) **Abstract**

Optically active 1,1-bis(alkylmethylphosphino)methanes represented by the following chemical formula (1): 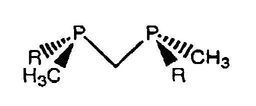 wherein "R" is a substituent selected from a group consisting of isopropyl group, cyclohexyl group, t-butyl group, and phenyl group are extremely small but exhibit extremely high stereoselectivity and catalytic activity.

## Description

### BACKGROUND OF THE INVENTION

### Field of the Invention

The present invention deals with the synthesis of novel optically active bisphosphinomethanes and use of their rhodium complexes or copper complexes in catalytic asymmetric syntheses.

### Description of the Related Art

Organic reactions, which use a metal complex as a catalyst, have been known for many years. Among such synthetic reactions, catalytic asymmetric syntheses of optically active compounds with the use of a chiral compound as a ligand have been considered to be especially useful and thus, various researches have been conducted in this field. For example, as reported by Ryoji Noyori in *Asymmetric Synthesis in Organic Synthesis* or by Iwao Ojima in *Catalytic Asymmetric Synthesis,* optically active phosphine compounds having various structures have been explored in order to improve catalytic activities.

Recently, a great number of optically active compounds have been used, as pharmaceutically active compounds (medical treatment), agricultural chemicals, and industrially important materials, and a practical method for catalytic asymmetric synthesis of optically active compounds is desired. Metal complexes are useful as asymmetric catalysts in asymmetric reactions. It is known that the catalytic activity largely depends on the structure of a phosphine compound which is used as an asymmetric ligand. 1,1-Bisphosphines form strained four-membered chelate complexes with transition metals, and such complexes can be employed as efficient catalysts for synthetic reactions.

In many of chiral ligands, the asymmetry of the skeleton carbon atom induces inequivalency of two phenyl groups on the phosphorus atom, thereby constructing an asymmetric environment. It is considered, however, that a ligand in which a phosphorus atom itself is asymmetric (i.e. a P-chiral phosphine) can lead to higher selectively.

To date, however, there have been reported only a few P-chiral phosphines such as 1,2 - bis[(*o*-methoxyphenyl)phenylphosphino]ethane (DIPAMP). This is due to the fact that there is no established method for synthesizing such phosphines in an optically pure form.

For the purpose of providing high catalytic activity in asymmetric synthesis as described above, enormous number of optically active diphosphine ligands have been developed to date. However, these diphosphines have not been sufficient in selectivity or catalytic activity to some kind of substrates. Further, no optically active 1,1-bisphosphine having a high asymmetry-recognition ability is known to date.

### SUMMARY OF THE INVENTION

It is, therefore, an object of the present invention to provide a novel diphosphine ligand which is much smaller than the previously reported ones but exhibits extremely high stereoselectivity and catalytic activity. Another object of the present invention is to provide rhodium and copper complexes of the diphosphine ligand in order to explore highly efficient catalytic asymmetric reactions.

To accomplish the above-mentioned objects, the inventors designed new optically active 1,1-bisphosphine ligands, which are represented by the following chemical formula (1). An important characteristic feature of these ligands is that they possess their chiral center at phosphorus, and another feature is that they are extremely small but are expected to exhibit excellent stereo-selectivity and catalytic activity when compared with the known diphosphine ligands. As a result of such finding, the present invention has been accomplished.

Accordingly, the present invention provides an optically active 1,1-bis(alkylmethylphosphino)methanes represented by the following chemical formula (1): 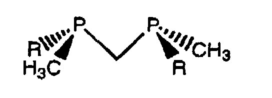 wherein "R" is a substituent selected from a group consisting of isopropyl group, cyclohexyl group, t-butyl group and phenyl group.

The present invention also provides rhodium complexes having such optically active 1,1- bis(alkylmethylphosphino)methanes as the ligands.

The present invention further provides copper complexes having such optically active 1,1- bis(alkylmethylphosphino)methanes as the ligands.

The present invention also provides organophosphorus compounds represented by the following chemical formula (2): 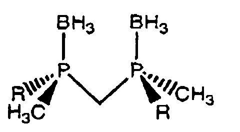 wherein "R" is a substituent selected from a group consisting of isopropyl group, cyclohexyl group, t-butyl group, and phenyl group.

The present invention further provides a catalytic asymmetric hydrogenation for the preparation of optically active saturated carboxylic acids or esters by the use of the above-mentioned rhodium complexes. 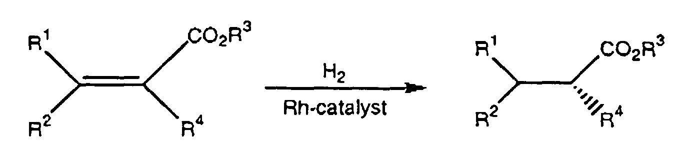 In the equation, "R¹", "R²" and "R³" are the same or different substituent selected from a group consisting of hydrogen atom, alkyl group, and aryl group. "R⁴" is a substituent selected from a group consisting of alkyl group, aryl group, CH₂CO₂R⁵, in which "R⁵" is a substituent selected from a group consisting of hydrogen atom, alkyl group, and aryl group, and NHR⁶, in which "R⁶" is a substituent selected from a group consisting of formyl group, alkyl group, and aryl group, or an ester of carboxylic acid.

The present invention further provides a Rh-catalyzed asymmetric hydrosilylation of ketones for the preparation of optically active secondary alcohols represented by the following chemical formula (3): 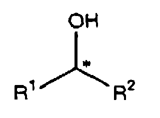 wherein "R¹" and "R²" are an alkyl group or an aryl group and different from each other.

The present invention still further provides a copper-catalyzed Michael addition of diethylzinc to α,β-unsaturated ketones for the preparation of optically active ketones.

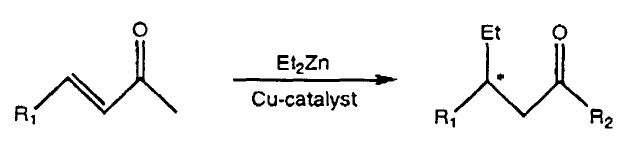

### BRIEF DESCRIPTION OF DRAWINGS

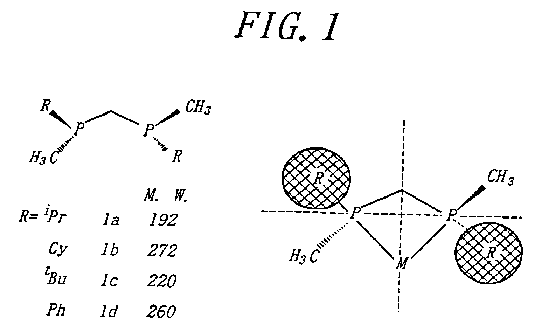

Fig. 1 is a schematic figure showing an image (conception) of the extremely small, optically active bisphosphine ligands according to the present invention.

### DETAILED DESCRIPTION OF THE PREFERRED EMBODIMENTS

The present invention will be described in detail with reference to preferred embodiments.

Bisphosphinomethanes according to the present invention are represented by the following chemical formula (4): 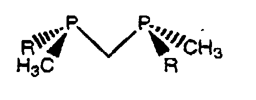 wherein "R" is a substituent selected from a group consisting of isopropyl group, cyclohexyl group, t-butyl group and phenyl group.

These novel optically active 1, 1 - bis(alkylmethyl phosphino)methanes have the C₂-symmetry, and they possess methyl group, which is the smallest alkyl group, and a bulky alkyl group such as t-butyl at each phosphorus atom. When these ligands form chelates with a metal, the resultant chelates are rigid four-membered ones, thereby constructing an asymmetric environment which may lead to highly enantioselective reactions.

The optically active bis(phosphino)methanes can be prepared through a process represented in the following scheme.

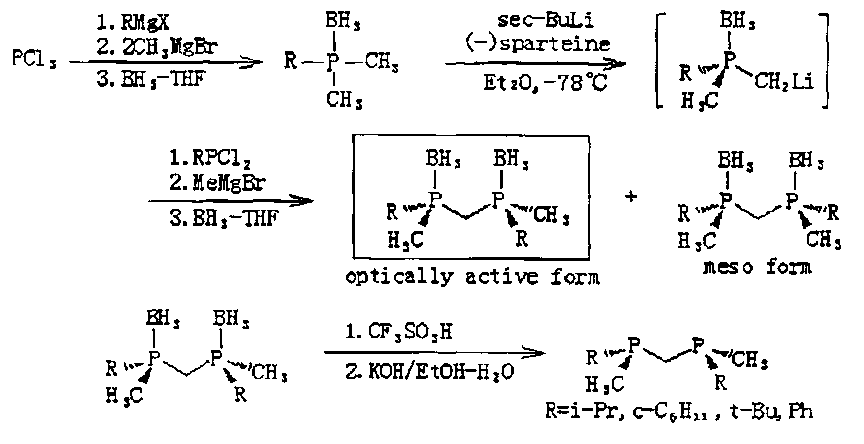

Namely, the key intermediates, optically pure 1,1-bis(phosphino)methane-borone complexes, are prepared according to the methods described in Journal of American Chemical Society, vol. 117 pp. 9075 - 9076 written by D. A. Evans et al. or Journal of American Chemical Society, vol. 120 pp. 1635 - 1636 written by Imamoto et al. The two boranato groups of the intermediates are removed by the method described in Tetrahedron Letters, vol. 35 pp. 9319 - 9322 written by T. Livinghouse et al.

More specifically, phosphorous trichloride is reacted successively with alkylmagnesium chloride, 2 equivalent amount of methylmagnesium bromide, and borane-tetrahydrofuran complex to yield an alkyldimethylphosphine-borane. In the presence of (-)- sparteine, the thus-obtained alkyldimethylphosphine-borane is subjected to enantionselective deprotonation of hydrogens at prochiral methyl groups with the use of *sec*-butylithium, followed by successive reactions with alkyldichlorophosphine, methylmagnesium bromide and borane-tetrahydrofuran complex. In this synthetic procedure, meso bis(phosphine)-boranes are also produced at the same time. These meso isomers, however, can be removed by recrystallization from methanol or ethanol. The thus-obtained optically pure intermediates are successively treated with trifluoromethanesulfonic acid and potassium hydroxide to yield optically pure diphosphine ligands. These phosphine ligands are small, having a molecular weight of 200 or so. Accordingly, the inventors denominated these bisphosphines as "MiniPHOS".

Rhodium complexes of the diphosphines thus obtained can be prepared by, for instance, a method described in a series of *Experimental Chemistry,* the fourth edition, vol. 18 pp. 327 - 353 (edited by Japan Chemistry Society, Maruzen). The preparation of rhodium complex is not limited to the above method and any known method may be employed. More specifically, the complexes consisting of rhodium and diphosphine compound in a 1:2 ratio can been obtained by stirring a mixture of the diphosphins and [Rh(nbd)₂]⁺X⁻ (X = BF₃ or PF₆) in tetrahydrofuran and then distilling off the solvent, followed by purification.

The steric structure of these complexes constructs an ideal asymmetric environment, where the first and third quadrants are occupied by the small methyl groups and the second and fourth quadrants are occupied by the bulky t-butyl groups.

Methods for preparing saturated carboxylic acids or esters by asymmetric hydrogenation of unsaturated carboxylic acids or esters with use of the rhodium complexes axe not limited to certain specific methods, and conventional practices may be employed. For example, such preparation can be conducted by introducing an unsaturated carboxylic acid or an ester, the rhodium complex and methanol into a pressure resistant container in an argon atmosphere and then infilling it with hydrogen. The thus-obtained saturated carboxylic acids or esters thereof have the optical purity of 88-100%. Also, the rhodium complex of the present invention can be used for reduction (asymmetric hydrogenation) of dehydroamino acids or itaconic acid, and there can be obtained reduction products having the optical purity of 88-100%. The isolated rhodium complexes may preferably be used, but it is also possible to simultaneously synthesize such rhodium complexes in situ just before use.

Methods for preparing optically active secondary alcohols by asymmetric hydrosilylation of simple ketones using the rhodium complexes as the catalysts are not limited to certain specific methods, and conventional practices may be employed. For instance, an optically active secondary alcohol can be obtained by stirring simple ketone, the rhodium complex and 1-naphthylphenylsilane in tetrahydrofuran.

Most of conventional, optically active bisphosphine ligands are not effective for asymmetric hydrosilylation of ketones, and only a few of them such as 2, 2''-bis[1-(dialkylphosphino)ethyl]-1,1''- biferrocene (TRAP) exhibit a selectivity exceeding 90%. On the other hand, the rhodium complex according to the present invention is quite effective in the asymmetric hydrosilylation of ketones, and thus, corresponding optically active alcohols can be obtained at a selectivity of 90% or more. Even in the case of dialkyl ketones, the reactions proceed in an asymmetric yield around 80%. Just like in the case of asymmetric hydrogenation, the rhodium complexes are preferably used in isolated state in the asymmetric hydrosilylation, but it is also possible to prepare the rhodium complexes in situ for use in the reaction system.

Methods for preparing optically active saturated ketones by asymmetric Michael reaction of diethylzinc with α,β - unsaturated ketones using copper complexes as the catalysts are not limited to certain specific methods, and conventional practices may be employed as such methods. For example, a saturated ketone can be obtained by stirring a mixture α,β - unsaturated ketone, copper complex, and diethylzinc in toluene. It is preferable to prepare the copper complex in the reaction system by allowing to react copper(II) trifluoromethanesulfonate with the diphosphines.

The Michael reaction has been one of the carbon-carbon bond-forming reactions of great significance in synthetic organic chemistry. Recently it has been emerged that the asymmetric Michael reaction of diethylzinc with cyclic enones proceeds in higher yield and higher stereoselectivity when copper-chiral phosphine complexes are used as the catalysts. The chiral phosphines used in such reactions are monodentate or hybrid ligands, whereas conventional optically active bisphosphine ligands such as BINAP, DuPHOS, or CHIRAPHOS result in extremely low asymmetric yield. On the other hand, the Michael reaction proceeds smoothly when it is conducted using 1 mmol% of the copper complex (diphosphine/Cu(OTf)₂ catalyst) at -80°C, the corresponding Michael adducts can be obtained at an yield as high as 97%.

The present invention will be hereinafter explained in further detail by way of the following examples. In this connection, the following examples are only given in illustration of the present invention and are not intended as limitations thereof.

### Example 1

### Synthesis of 1, 1 - bis [(t-butyl)dimethylphosphino] methane-borane

In an argon atmosphere, (-)- spartein (24 mmol, 5.64 g) dissolved in 20 mL of diethyl ether was cooled to -78°C, and 25 mL of *sec* - BuLi (0.97 M of cyclohexane solution, 24 mmol) was added to the solution. After 10 minutes, t-butyldimethylphosphine-borane (20 mmol, 2.64 g) was added in the form of powder, and the resulting solution was stirred for 3 hours at -78°C. Then, t-butyldichlorophosphine (20 mmol, 3.18 g) in diethylether (20 mL) was added dropwise at -78°C. The resulting reaction solution was gradually warmed to room temperature, and then cooled to 0 °C. Then, 20 mL of methylmagnesium bromide (1.0 M of THF solution, 20 mmol) was slowly added. After again warming this reaction mixture to room temperature and then cooling the temperature to 0°C, 70mL of tetrahydrofuran solution (1.0 M, 70 mmol) of borane - tetrahydrofuran complex was added. The reaction mixture was treated with water (about 100 mL) containing ethyl acetate (about 20 mL). The organic portion was separated and the aqueous portion was extracted with ethyl acetate. The organic layer was collected and washed with saturated NaCl solution, and dried over sodium sulfate. The solution was concentrated under the reduced pressure and the residue was purified by column chromatography. The obtained crystals were recrystallized from methanol to give optically pure (*S*, *S*) - 1, 1 - bis [(t-butyl)dimethylphosphino] methane - borane (1.1 g, yield: 22%).

The solid state properties thereof were as follows:
Melting point 188.0 - 190.0°C; [α]¹⁴_{D} - 4.7° (c 0.93, CHCl₃); ¹HNMR(400 MHz, CDCL₃) δ 0.20 - 1.04 (m, 6H), 1.18 (d, J = 13.9 Hz, 18H), 1.54 (d, J = 10.0 Hz, 6H), 1.77 (t, 1H); ¹³CNMR (100 MHz, CDCl₃) δ 7.2 (d, J = 33.6 Hz), 13.4(t), 25.2 (d, J = 2.5Hz), 29.4(d, J = 4.9 Hz), 29.8(d, J = 4.1 Hz); IR(KBr) 2980, 2400, 1460, 1170, 910cm⁻¹, HRMS calcd for C₁₁H₂₉P₂B₂ 245. 1935, found 245. 1932.

### Example 2 - 4

(*S, S*)-1,1-Bis [(isopropyl)dimethylphosphino] methane- borane, (*S, S*)-1,1-bis [(cyclohexyl)dimethylphosphino) methane - borane and (*S, S*)- 1,1-bis [(phenyl)dimethylphosphino] methane - borane were synthesized in the same manner as in example 1.

The solid state properties thereof were as follows.

(*S, S*)- ,1-Bis [(isopropyl)dimethylphosphino] methane-borane: Melting point 105.0 - 106.0°C; [α]¹⁶_{D} - 1.0° (c 0.87, CHCl₃); ¹HNMR (400 MHz, CDCL₃) δ 0.36 -0.68 (m, 6H), 1.16-1.21 (q, 12H), 1.49 (d, 6H), 1.49 (d, 6H, J = 10.1 Hz), 1.82 (t, 2H); 2.02 - 2.12 (m, 2H); ¹³CNMR (100 MHz, CDCl₃) δ 8.7 (d, J = 35.3 Hz), 16.2 (d, 12.3 Hz), 16.4 (t), 26.0 (dd, J = 4.1 Hz, J = 39.4 Hz); IR(KBr) 2980, 2380, 1460, 1070, 920cm⁻¹, HRMS calcd for C₉H₂₅P₂B₂ 217. 1621, found 217. 1630.

(*S, S*)-1,1-Bis [(cyclohexyl)dimethylphosphino] methane- borane: Melting point 135.0 - 136.5°C; [α]¹⁶_{D} - 5.7° (c 0.96, CHCl₃); ¹HNMR(400 MHz, CDCL₃) δ 0.07-0.85 (m, 6H), 1.17 - 1.41 (m, 14H), 1.46 (d, 6H, J = 10.1 Hz), 1.74 - 1.88 (m, 14H); ¹³CNMR (100 MHz, CDCl₃) δ 8.7 (d, J = 35.3 Hz), 16.3 (t), 25.8 (d, J = 5.7 Hz), 26.0, 26.3 (d, J = 3.3 Hz), 26.5 (d, J = 2.5 Hz), 35.6 (dd, J = 38.6 Hz, J = 3.3 Hz); IR(KBr) 2940, 2380, 1450, 1170, 920cm⁻¹, HRMS calcd for C₁₅H₃₃P₂B₂ 297. 2249, found 297. 2198.

(*S, S*)-1,1-Bis [(phenyl)dimethylphosphino] methane-borane: Melting point 150.0 - 152.0°C; [α]²⁰_{D} - 5.1°(c 1.28, CHCl₃); ¹HNMR(400 MHz, CDCL₃) δ 0.55 - 1.26 (m, 6H), 1.84 (d, 6H, J = 10.1 Hz), 2.41 (t, 2H), 7.27 -7.56 (m, 10H); ¹³CNMR (100 MHz, CDCl₃) δ 12.7 (d, J = 35.3 Hz), 27.1 (t), 128.7 (d, J = 9.8 Hz), 131.2 (d, J = 9.8 Hz), 131.2 (d, J = 9.8 Hz), 131.5; IR(KBr) 3050, 2940, 2380, 1440, 1110, 920cm⁻¹, HRMS calcd for C₁₅H₂₁P₂B₂ 285. 1310, found 285. 1317.

### Example 5

### Synthesis of (S, S) - 1, 1 - bis [(t-butyl)dimethylphosphino] methane

In an argon atmosphere, a toluene solution (1 mL) of optically pure 1,1-bis [(t-butyl)dimethylphosphino] methane- borane (0.2 mmol, 0.055 g) was cooled to 0 °C, and trifluoromethanesulfonic acid (1.0 mmol, 80 µl) was added. After warming to room temperature and keeping for 1 hour, 10% potassium hydroxide ethanol solution was added and the resulting solution was stirred at 50 °C for 2 hours. The reaction mixture was extracted with diethyl ether, and the extracts were dried over sodium sulfate. The solvent was removed on an evaporator and the residue was purified by column chromatography on alumina to give the desired diphosphine as transparent liquid. (0.044 g, yield: 100%)

(*S, S*) - 1,1-bis [(isopropyl)dimethylphosphino] methane, (*S, S*) - 1,1-bis [(cyclohexyl)dimethylphosphino]methane and (*S, S*) - 1,1 - bis [(phenyl)dimethylphosphino)methane were also synthesized in the same manner.

### Example 6

### Synthesis of rhodium complex and asymmetric hydrogenation

In an argon atmosphere, bis (bicyclo[2, 2, 1]hepta - 2, 5 - diene)rhodium (I) tetrafluoroborate (0.1 mmol, 0.037 g) was reacted with (*S, S*)-1,1-bis [(t-butyl)dimethylphosphino]methane (0.1 mmol, 0.044 g) in a tetrahydrofuran solution (10 mL), and there obtained a reaction solution (a solution of rhodium complex). Another reactor was charged with 2-acetamidacrylic acid (1 mmol, 0.13 g) and 5 mL of methanol, and filled with hydrogen. Then, 0.2 mL of the above reaction solution (the solution of rhodium complex) was added to the reactor. This reaction was conducted at room temperature under 1 atm of hydrogenation pressure. After 20 hours, the resulting reaction solution was subjected to capillary gas chromatography (column: Lipodex A)) to analyze the products, and it was confirmed that almost pure products (> 99.9%ee) were obtained quantitatively.

### Example 7

### Asymmetric hydrosilylation by the use of rhodium complex

In an argon atmosphere, 1-acetonaphthone (1 mmol, 0.17 g), 1-naphthylphenylsilane (1.5 mmol, 0.35 g) and 1 mL of tetrahydrofuran were placed into a flask which was cooled to -40°C and then, 1 mL of a solution of rhodium complex, which was obtained in Example 6, was added. After 80 hours, 1 M hydrochloric acid was added to this reaction solution. Organic layer was separated and the aqueous layer was extracted with ethyl acetate. The combined extracts were washed with saturated NaCl solution and dried over sodium sulfate. After evaporation of the solvent under the reduced pressure, the residue was purified by column chromatography to afford 1-(1' -naphtyl)-1-ethanol (0.16 g, yield: 90%). The optical purity of the products analyzed by HPLC (column: Chiralcel OJ manufactured by Daisel Corp.) was 97%ee.

### Example 8

### Asymmetric Michael reaction by the use of copper complex

In an argon atmosphere, copper(II) trifluoromethanesulfonate (0.05 mmol, 0.018 g) and (*S, S*) - 1, 1 - bis [(t-butyl)dimethylphosphino]methane (0.05 mmol, 0.011 g) were reacted in toluene (5 mL) at room temperature.

This solution was cooled to -80°C, and 2-cyclohepten-1-on (5 mmol, 0.055 g) together with 6 mL of diethylzinc (1M toluene solution, 6 mmol) were added.

After 24 hours, 1M hydrochloric acid was added to this reaction. The mixture was extracted with ethyl acetate three times, and the combined extracts were washed with saturated NaCl solution and dried over sodium sulfate. The solvent was removed on an evaporator and the residue was purified by column chromatography to afford 3-ethyl-1-cycloheptane (0.64 g, yield: 91%). The optical purity of the product analyzed by capillary gas chromatography (column: Lipodex A) was 97%ee.

As described above, the optically active bisphosphinomethanes synthesized in this invention are the smallest among those reported so far. The rhodium complexes are effective as catalysts for the asymmetric hydrogenation and asymmetric hydrosilylation, and copper complexes are effective as catalysts for the asymmetric Michael reaction. The rhodium complexes and copper complexes exhibit high to excellent stereoselectivity and catalytic activity when compared with the complexes of so far reported P-chiral phosphines. In addition, chiral amino acids and alcohols are obtained in high optical purity by the use of rhodium complexes and copper complexes of the bisphosphinomethanes. As a result, many important optically active compounds such as pharmaceutical active compounds, and agrochemicals can be easily synthesized according to the present invention.

Although the invention has been described with reference to specific preferred embodiments, they were given by way of examples only. Therefore, it should be noted that various changes and modifications may be made on them without departing from the scope of the present invention as defined by the appended claims.

## Claims

1. Optically active 1, 1-bis(alkylmethylphosphino) methanes represented by the following chemical formula (1): 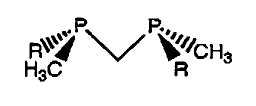 wherein "R" is a substituent selected from a group consisting of isopropyl group, cyclohexyl group, t-butyl group and phenyl group.

2. Rhodium complexes with the optically active 1,1-bis(alkylmethylphosphino)methanes as set forth in claim 1 as a ligand.

3. Copper complexes with the optically active 1,1-bis(alkylmethylphosphino)methanes as set forth in claim 1 as a ligand.

4. Organophosphorus compound represented by the following chemical formula (2): 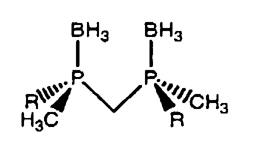 wherein "R" is a substituent selected from a group consisting of isopropyl group, cyclohexyl group, t-butyl group, and phenyl group.

5. Catalytic asymmetric hydrogenation for the preparation of optically active saturated carboxylic acids or esters represented by the following scheme: 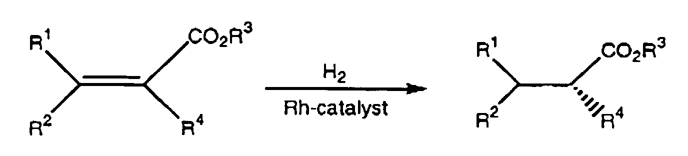 wherein "R¹", "R²" and "R³" are the same or different substituent selected from a group consisting of hydrogen atom, alkyl group, and aryl group, and "R⁴" is a substituent selected from a group consisting of alkyl group, aryl group, CH₂CO₂R⁵, in which "R⁵" is a substituent selected from a group consisting of hydrogen atom, alkyl group, and aryl group, and NHR⁶, in which "R⁶" is a substituent selected from a group consisting of formyl group, alkyl group, and aryl group, or an ester of carboxylic acid.

6. Rh-catalyzed asymmetric hydrosilylation of ketones for the preparation of an optically active secondary alcohols represented by the following chemical formula (3): 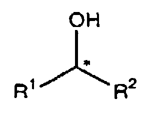 wherein "R¹" and "R²" are an alkyl group or an aryl group and different from each other.

7. Copper-catalyzed Michael addition of diethylzinc to α,β-unsaturated ketones for the preparation of optically active ketones represented by the following scheme: 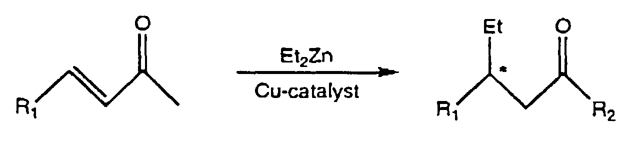 wherein "R¹" and "R²" are an alkyl group or an aryl group and different from each other.
